# EUROPÄISCHE PATENTSCHRIFT

(11) **EP 3 944 867 B1**
(45) Veröffentlichungstag und Bekanntmachung des Hinweises auf die Patenterteilung: **22.02.2023**
(21) Anmeldenummer: 20188944.1
(22) Anmeldetag: 31.07.2020
(51) Int. Cl.: A61L 2/28, A61L 2/20, C12Q 1/04, C12Q 1/22, G01N 1/40, G01F 15/00, G21F 5/002

(54) **ANLAGE UND VERFAHREN ZUR STERILITÄTSPRÜFUNG VON RADIOAKTIVEN STOFFEN**
SYSTEM AND METHOD FOR TESTING THE STERILITY OF RADIOACTIVE MATERIALS
INSTALLATION ET PROCÉDÉ DE VÉRIFICATION DE LA STÉRILITÉ DE SUBSTANCES RADIOACTIVES

(43) Veröffentlichungstag der Anmeldung: 02.02.2022
(73) Patentinhaber: CUP Laboratorien Dr. Freitag GmbH, 01454 Radeberg (DE)
(72) Erfinder: KNOLLE, Stefan, 01454 Radeberg (DE); PROTZE, Severine, 01454 Radeberg (DE); JANSEN, Sven, 02692 Großpostwitz (DE)
(74) Vertreter: Kailuweit & Uhlemann Patentanwälte Partnerschaft mbB

(56) Entgegenhaltungen:
- EP-A1- 2 918 684
- WO-A1-2012/092394
- WO-A2-2017/192191
- DE-A1- 3 012 085

## Beschreibung

Die Erfindung betrifft eine Anlage zur Sterilitätsprüfung von radioaktiven Stoffen, die Verwendung der Anlage zur Sterilitätsprüfung von radioaktiven Stoffen, bevorzugt radioaktiven Pharmazeutika und/oder Diagnostika, sowie ein Verfahren zur Sterilitätsprüfung von radioaktiven Stoffen.

### Stand der Technik

Ein Teil der Qualitätskontrolle von unter aseptischen Bedingungen hergestellten Arzneimitteln und Medizinprodukten für die Anwendung an Mensch oder Tier stellt die Sterilitätsprüfung gemäß Europäischen Arzneibuch (Ph. Eur. 2.6.1 - Prüfung auf Sterilität) oder U.S Pharmacopeia (USP <71>) dar.

Arzneimittel und Medizinprodukte umfassen wässrige Lösungen, lösliche Pulver, Öle und ölige Lösungen, Salben und Cremes, Infusionsbeutel, Implantate, z.B. Herzklappen oder Stents; und chirurgisches Nahtmaterial.

Entscheidend für die Sterilitätsprüfung von Arzneimitteln und Medizinprodukten ist die Einhaltung aseptischer Prüfbedingungen, was häufig durch den Einsatz eines Isolators (Sterilbox) zur Kontaminationsvermeidung gewährleistet wird.

Die Sterilitätsprüfung erfolgt bevorzugt im Membranfiltrations-Verfahren. Das Membranfiltrations-Verfahren ist ein Verfahren zur mechanischen Anreicherung von Mikroorganismen aus einer beliebigen Menge eines filtrierbaren Untersuchungsmaterials. Selbst bei minimalem Keimgehalt ermöglicht dieses Verfahren exakte Keimzahlbestimmungen. Dabei wird ein bestimmtes Volumen einer Flüssigkeit durch ein Membranfilter mit definierter Porenweite filtriert, wobei die darin enthaltenen Keime auf der Filteroberflache zurückgehalten werden. Der Filter wird anschließend auf einen Nährboden gebracht. Die Nährstoffe des Nährbodens ermöglichen das Heranwachsen der Einzelkeime zu diagnostizierbaren Kolonien.

Alternativ können die kontaminierten Filter in flüssige Nährmedien überführt werden. Eine Vermehrung von Mikroorganismen wird je nach Medium entweder durch Trübung oder, falls Indikatorfarbstoffe im Medium enthalten sind, durch Farbumschläge angezeigt.

WO 2017/192191 A2 offenbart ein System zum Transfer von Radionuklid-Generator-Säulen von einer Produktionslinie in eine Reinraumumgebung umfassend eine Strahlungseindämmungskammer, einen Isolator, der mit der Strahlungseindämmungskammer verbunden ist, eine rotierende Transfertür, die zwischen der Strahlungseindämmungskammer und dem Isolator angeordnet ist und einen Hohlraum zur Aufnahme einer Radionuklid-Generator-Säulenanordnung, und einen antimikrobiellen Dampferzeuger, der mit dem Isolator verbunden ist, wobei die Überführungstür so ausgelegt ist, dass sie sich dreht, während antimikrobieller Dampf durch den antimikrobiellen Dampfgenerator in den Isolator eingeführt wird. WO 2017/192191 A2 beschreibt die Membranfiltration oder die direkte Animpfung zur Sterilitätsprüfung von Elutionssäulen (Absatz [0044] und [0045]) sowie die Verwendung eines sterilen Kunststoffkanisters umfassend einen Sterilisationsfilter für die Membranfiltration. Insbesondere wird bei der Sterilitätsprüfung eine Säuleneinheit eluiert und die eluierte Produktflüssigkeit wird durch den Kunststoffkanister geleitet, der am Kanisterausgang einen Sterilisationsfilter enthält. Der Kanister wird dann mit einem geeigneten Wachstumsmedium gefüllt und bebrütet, um das Wachstum der vorhandenen Mikroorganismen zu fördern.

DE 30 12 085 A1 offenbart eine Vorrichtung zur Sterilitätsprüfung von Fluiden mit mindestens einer geschlossenen Büchse umfassend einen Einlass für das zu untersuchende Fluid, für eine Spüllösung und ein Nährmedium, einen Auslass und einen Membranfilter.

Die Weiterentwicklung der Membranfiltration zur Abtrennung von Mikroorganismen bzw. Zellen aus Lösungen betrifft insbesondere die Herstellung von neuartigen Filtermaterialien.

US 4,441,996 A offenbart eine Anlage zur Herstellung von Trinkwasser aus bakteriell verunreinigtem Kaltwasser mittels eines porösen Submikronfilters unter Druckzufuhr umfassend den porösen Submikronfilter, eine Aufnahmekammer und eine Öffnung zum sterilen Auslass des filtrierten Wassers.

US 2005/0098495 A1 offenbart ein Trennmaterial und ein Verfahren zur Reinigung von Fluiden, einschließlich Wasser oder Gase, von chemischen und mikrobiologischen Kontaminationen. Das Trennmaterial umfasst ein partikuläres Filtermaterial sowie ein erstes und ein zweites Bindemittel, wodurch ein poröses Filtermaterial mit einer nicht-porösen Beschichtung erhalten wird.

Im Falle von nicht filtrierbaren Lösungen wird das Arzneimittel oder Medizinprodukt in wässrigen oder öligen Lösungsmitteln gelöst bzw. verdünnt und anschließend filtriert. Dabei darf das verwendete Lösungsmittel die Prüfung auf Sterilität nicht negativ beeinflussen, insbesondere keine antimikrobiellen Eigenschaften aufweisen.

Im Falle von nicht löslichen Produkten (z.B. Implantate, chirurgisches Nahtmaterial) erfolgt die Prüfung im Direktbeschickungsverfahren, wobei das Produkt direkt in das Nährmedium eingebracht wird.

Ein Problem stellt bisher die Sterilitätsprüfung von radioaktiven Arzneimitteln dar.

Die Durchführung des Membranfiltrations-Verfahren ist aufgrund der Handhabung und Lagerung des radioaktiven Filtrats problematisch.

Daher erfolgt die Sterilitätsprüfung von radioaktiven Stoffen, insbesondere radioaktiven Pharmazeutika und/oder Diagnostika, häufig mittels des Direktbeschickungsverfahrens. Nachtteilig ist dabei die Prüfung auf eine geringe Probenmenge begrenzt, da die Radioaktivität für die Keimprüfung problematisch ist. Weiterhin entspricht die Sterilitätsprüfung von radioaktiven Pharmazeutika mittels Direktbeschickungsverfahren nicht den rechtlichen Vorgaben gemäß Europäischen Arzneibuch (Ph. Eur. 2.6.1 - Prüfung auf Sterilität) oder U.S Pharmacopeia (USP <71 >).

Alternativ, erfolgt die Prüfung der Sterilität von radioaktiven Stoffen nach dem Abklingen der Radioaktivität, insbesondere nach ca. drei Monaten, im Membranfilterverfahren.

Allerdings kann dieses Verfahren nur bei radioaktiven Stoffen mit kurzlebigen Nukliden durchgeführt werden. Langlebige Nuklide sind aufgrund des hohen zeitlichen Aufwands ausgeschlossen. Weiterhin nachteilig ist die Sterilitätsprüfung mehrere Monate nach der Herstellung der radioaktiven Stoffe wenig aussagekräftig, insbesondere aufgrund des negativen Einflusses der Radioaktivität auf das Keimwachstum.

Bekannte Verfahren zur Filtration radioaktiver Lösungen besitzen ausschließlich die Aufgabe, der Abtrennung der radioaktiven Verbindungen bzw. der selektiven Abtrennung bestimmter radioaktiver Verbindungen.

CN 10 7935 217 A offenbart ein System zur Abtrennung von Radon aus Wasser umfassend eine automatische Zufuhreinheit, einen unterirdischen Probentank, einen Rührmischbehälter, eine Keramikfiltereinheit und einen Abwassertank. Die Beförderung der Probe bzw. des zu reinigenden Wasser erfolgt mittels einer Pumpe. Weiterhin umfasst die Anlage eine Überdruckleitung.

JP 2013 007 634 A offenbart eine Wasserreinigungsvorrichtung zum Entfernen radioaktiver Substanzen aus Leitungswasser mittels einer Schicht aus Zeolith und/oder Neolith zum Entfernen von Cäsium und eine verwitterte körnige Granitschicht zum Entfernen von Chlor. Die Vorrichtung umfasst weiterhin einen Einlass, einen Probentank, eine Pumpe sowie eine Druckanzeige. Nachteilig können mittels dieser Vorrichtungen die radioaktiven Substanzen, nicht von ggf. vorhandenen Mikroorganismen abgetrennt werden.

Daher besteht die Aufgabe der vorliegenden Erfindung darin, eine Anlage und ein Verfahren zur Sterilitätsprüfung von radioaktiven Stoffen bereitzustellen.

Die Aufgabe wird durch die Merkmale der unabhängigen Ansprüche gelöst. Vorteilhafte Ausgestaltungen sind in den abhängigen Ansprüchen angegeben.

Erfindungsgemäß wird die Aufgabe gelöst durch eine Anlage zur Sterilitätsprüfung von radioaktiven Stoffen, umfassend
i. eine Filtratflasche aufweisend mindestens eine flüssigkeitsfördernde Leitung und mindestens eine Druckausgleichsleitung, und
ii. mindestens einen Transportwagen, wobei der Transportwagen geeignet ist, die Filtratflasche aufzunehmen,
   wobei der Transportwagen bewegbar ausgebildet ist,
   wobei mindestens die Filtratflasche von einer Abschirmung gegen ionisierende Strahlung umgeben ist,
iii. eine Vakuumpumpe,
   wobei die Vakuumpumpe an die flüssigkeitsfördernde Leitung angeschlossen und geeignet ist, Filtrat enthaltend den radioaktiven Stoff in die Filtratflasche zu pumpen,
iv. mindestens ein selbstabdichtendes Verbindungsstück, und
v. einen Isolator, wobei der Isolator mit der Filtratflasche auf dem Transportwagen über die mindestens eine flüssigkeitsfördernde Leitung und die mindestens eine Druckausgleichsleitung verbunden ist, wobei der Isolator eine Vorrichtung zur Membranfiltration umfasst,
wobei das mindestens eine selbstabdichtende Verbindungsstück die Filtratflasche über die flüssigkeitsfördernde Leitung und/oder die Druckausgleichsleitung mit dem Isolator verbindet.

### Kailuweit & Uhlemann I Patentanwälte

Vorteilhaft ist die erfindungsgemäße Anlage mit einem Isolator verbunden und kann mit einem Abklingbecken verbunden werden. Vorteilhaft ermöglicht die erfindungsgemäße Anlage die Membranfiltration eines radioaktiven Stoffes unter Schutz der Umgebung vor schädlichen Auswirkungen der zu bearbeitenden radioaktiven Stoffe, insbesondere einem Personenschutz, und somit die Sterilitätsprüfung der radioaktiven Stoffe.

Unter dem Begriff "radioaktiver Stoff" wird ein Reinstoff oder eine Zusammensetzung bzw. ein Gemisch von verschiedenen Stoffen umfassend mindestens ein radioaktives Element, wie ¹²³I oder ¹⁷⁷Lu; verstanden.

Unter dem Begriff "Filtratflasche" wird eine Flasche, z. B. eine Weithals-Chemikalienflasche, verstanden, welche mindestens eine zumindest flüssigkeitsdichte Durchführung für eine flüssigkeitsfördernde Leitung und mindestens eine weitere zumindest flüssigkeitsdichte Durchführung für eine Druckausgleichsleitung aufweist Erfindungsgemäß ist die Filtratflasche über die flüssigkeitsfördernde Leitung mit einer Vorrichtung zur Membranfiltration verbunden.

In Ausführungsformen ist die Filtratflasche aus Glas, insbesondere Borosilikatglas; Kunststoff und/oder Metall. Zweckmäßig ist die Filtratflasche formstabil gegenüber Über- und Unterdruck.

In Ausführungsformen weist die Filtratflasche ein Fassungsvermögen im Bereich von 200 ml bis 10 l auf, bevorzugt ein Fassungsvermögen im Bereich von 1 l bis 5 l, besonders bevorzugt ein Fassungsvermögen von 3 l.

Unter dem Begriff "Druckausgleichsleitung" wird eine Leitung zum Ausgleich des Gasdrucks zwischen der Filtratflasche und einem Isolator oder einer Lüftungsanlage, insbesondere einem *laminar air flow system;* verstanden.

In weiteren Ausführungsformen ist die flüssigkeitsfördernde Leitung und die mindestens eine Druckausgleichsleitung eine gegenüber Über- und Unterdruck stabile Leitung, bevorzugt aus Gummi und/oder Metall, besonders bevorzugt aus Edelstahl.

In Ausführungsformen weist die Filtratflasche einen Schraubverschlussdeckel auf, der mindestens eine Durchführung für die flüssigkeitsfördernde Leitung und mindestens eine Durchführung für die Druckausgleichsleitung aufweist

Unter dem Begriff "Transportwagen" wird ein bewegbarer Wagen verstanden, welcher fähig ist, mindestens die Filtratflasche räumlich zu bewegen, bevorzugt von einem Isolator zu einem Abklingbecken und/oder von einem Abklingbecken zu einem Isolator.

In Ausführungsformen weist der Transportwagen Rollen oder Räder an der Unterseite auf, bevorzugt mindestens zwei Rollen, besonders bevorzugt vier Rollen.

Unter dem Begriff "Abschirmung gegen ionisierende Strahlung" wird ein Gehäuse verstanden, welches fähig ist, die Strahlenintensität ionisierender Strahlung eines radioaktiven Stoffes, insbesondere der Alpha-, Beta- und Gammastrahlung, außerhalb des Gehäuses um mindestens 90 % zu reduzieren, bevorzugt um 100 % zu reduzieren.

In Ausführungsformen ist die Abschirmung gegen ionisierende Strahlung an mindestens einer Längsseite der Filtratflasche angebracht.

Bevorzugt umschließt die Abschirmung gegen ionisierende Strahlung die Filtratflasche vollständig.

In Ausführungsformen weist die Abschirmung gegen ionisierende Strahlung ein Metall mit einer Dicke von mindestens 40 mm, bevorzugt mit einer Dicke ein Metall mit einer Dicke, besonders bevorzugt mit einer Dicke im Bereich von 40 mm bis 50 mm; auf. Zweckmäßig ist die Dicke abhängig von der Auswahl des Metalls.

In Ausführungsformen ist das Metall in der Abschirmung gegen ionisierende Strahlung aus Blei, Edelstahl, Stahl, Kupfer, Wolfram oder Uran, besonders bevorzugt aus Blei, Edelstahl und Stahl; ausgewählt.

In weiteren Ausführungsformen umfasst die Abschirmung gegen ionisierende Strahlung weiterhin eine Plexiglasschicht.

Vorteilhaft wird durch die Abschirmung gegen ionisierende Strahlung ein Personenschutz gewährleistet.

Erfindungsgemäß umfasst die erfindungsgemäße Anlage weiterhin eine Vakuumpumpe, wobei die Vakuumpumpe geeignet ist, Filtrat enthaltend die radioaktiven Stoffe in die Filtratflasche zu pumpen. Erfindungsgemäß ist die Vakuumpumpe an die flüssigkeitsfördernde Leitung angeschlossen. Vorteilhaft entsteht durch die Vakuumpumpe ein Unterdruck zumindest in der flüssigkeitsfördernden Leitung, wodurch kein Austritt des Filtrats enthaltend die radioaktiven Stoffe möglich ist.

In Ausführungsformen weist die Filtratflasche einen Flüssigkeitsfüllstandmesser auf, wobei der Flüssigkeitsfüllstandmesser ein optisches und/oder akustisches Signal bei einem vordefinierten Füllstand des Filtrats abgibt. In Ausführungsformen gibt Flüssigkeitsfüllstandmesser ein optisches und/oder akustisches Signal bei einem Füllstand des Filtrats im Bereich von 60 % (v/v) bis 90 % (v/v) in Bezug auf das maximale Füllvolumen, bevorzugt bei einem Füllstand des Filtrats von 80 % (v/v) in Bezug auf das maximale Füllvolumen ab.

Vorteilhaft gibt der Flüssigkeitsfüllstandmesser ein optisches und/oder akustisches Signal bei einem vordefinierten Füllstand des Filtrats, bis zu einer Unterbrechung der Stromzufuhr der Vakuumpumpe.

Erfindungsgemäß umfasst die erfindungsgemäße Anlage weiterhin mindestens ein selbstabdichtendes Verbindungsstück, welches mit einem Isolator verbunden ist. Erfindungsgemäß verbindet das mindestens eine selbstabdichtende Verbindungsstück die Filtratflasche über die flüssigkeitsfördernde Leitung und/oder die Druckausgleichsleitung mit dem Isolator. Zweckmäßig kann das mindestens eine selbstabdichtende Verbindungsstück die Filtratflasche, insbesondere über die flüssigkeitsfördernde Leitung und/oder die Druckausgleichsleitung; mit einem Abklingbecken verbunden werden.

In Ausführungsformen weist das selbstabdichtendende Verbindungsstück eine Rohrverschraubungseinrichtung, z.B. eine Klemmverschraubung oder Klemmringverschraubung oder Schneidringverschraubung oder Bördelverschraubung; auf. Vorteilhaft ermöglichen die selbstabdichtenden Verbindungsstücke ein tropffreies Trennen der Filtratflasche von einem Isolator und/oder dem Abklingbecken.

Erfindungsgemäß umfasst die erfindungsgemäße Anlage weiterhin einen Isolator, wobei der Isolator mit der Filtratflasche auf dem Transportwagen über die mindestens eine flüssigkeitsfördernde Leitung und die mindestens eine Druckausgleichsleitung verbunden ist. Vorteilhaft wird durch den Isolator die Umgebung vor schädlichen Auswirkungen der zu bearbeitenden radioaktiven Stoffe geschützt, insbesondere ein Personenschutz ermöglicht.

In bevorzugten Ausführungsformen ist der Isolator eine Sterilbox. Unter dem Begriff "Sterilbox" oder auch "Handschuhbox oder Handschuhkasten" wird eine Anlage verstanden, welche gegenüber dem umgebenden Arbeitsraum hermetisch und gasdicht abgeschlossen ist. Innerhalb der Sterilbox kann eine definierte Atmosphäre zur Bearbeitung radioaktiver Stoffe erzeugt werden.

In Ausführungsformen ist an mindestens einer Seite des Isolators eine Lexan- oder Glasscheiben vorhanden, wobei durch die Lexan- oder Glasscheibe der Innenraum einsehbar ist.

In Ausführungsformen umfasst der Isolator weiterhin Edelstahl.

In Ausführungsformen weist der Isolator mindestens zwei Durchführungen auf, welche mittels Gummi- oder Kunststoffhandschuhen ein Hineinreichen in die Anlage ermöglichen.

In Ausführungsformen weist der Isolator mindestens eine evakuierbare Kammer oder Schleuse auf.

In Ausführungsformen umfasst der Isolator weiterhin einen Gaseinlass, wobei der Gaseinlass für die Zufuhr von Inertgas und/oder von Wasserstoffperoxid zur Sterilisierung des Isolators ausgebildet ist.

In Ausführungsformen istdie Verbindung zwischen dem Isolator und/oder der Abpumpvorrichtung mit der Filtratflasche auf dem Transportwagen als Strahlenfalle ausgeführt.

Zweckmäßig ist die Durchführung der flüssigkeitsfördernden Leitung und/oder der Druckausgleichsleitung durch die Abschirmung gegen ionisierende Strahlung als Strahlenfalle ausgeführt.

Erfindungsgemäß umfasst der Isolator eine Vorrichtung zur Membranfiltration.

In Ausführungsformen umfasst die Vorrichtung zur Membranfiltration mindestens einen Membranfilter aus Celluloseacetat oder Cellulosenitrat und/oder aufweisend Poren im Bereich von 0,2 µm bis 0,45 µm.

In Ausführungsformen weist die Vorrichtung zur Membranfiltration ein Fassungsvermögen im Bereich von 50 ml bis 250 ml, bevorzugt ein Fassungsvermögen von 100 ml auf.

Ein weiterer Aspekt der Erfindung betrifft die Verwendung einer erfindungsgemäßen Anlage zur Sterilitätsprüfung von radioaktiven Stoffen, bevorzugt radioaktiven Pharmazeutika und/oder Diagnostika, wie Luthatera^{®} oder loflupan^{®}. Bevorzugt erfolgt die Sterilitätsprüfung von radioaktiven Stoffen mittels der erfindungsgemäßen Anlage nach Europäischen Arzneibuch (Ph. Eur. 2.6.1 - Prüfung auf Sterilität) oder U.S Pharmacopeia (USP <71>).

In Ausführungsformen erfolgt die Verwendung einer Anlage umfassend
i. eine Filtratflasche aufweisend mindestens eine flüssigkeitsfördernde Leitung und mindestens eine Druckausgleichsleitung, und
ii. mindestens einen Transportwagen, wobei der Transportwagen geeignet ist, die Filtratflasche aufzunehmen,
   wobei der Transportwagen bewegbar ausgebildet ist,
   wobei mindestens die Filtratflasche von einer Abschirmung gegen ionisierende Strahlung umgeben ist,
iii. eine Vakuumpumpe,
   wobei die Vakuumpumpe an die flüssigkeitsfördernde Leitung angeschlossen und geeignet ist, Filtrat enthaltend den radioaktiven Stoff in die Filtratflasche zu pumpen,
iv. mindestens ein selbstabdichtendes Verbindungsstück, und
v. einen Isolator, wobei der Isolator mit der Filtratflasche auf dem Transportwagen über die mindestens eine flüssigkeitsfördernde Leitung und die mindestens eine Druckausgleichsleitung verbunden ist, wobei der Isolator eine Vorrichtung zur Membranfiltration umfasst,
wobei das mindestens eine selbstabdichtende Verbindungsstück die Filtratflasche über die flüssigkeitsfördernde Leitung und/oder die Druckausgleichsleitung mit dem Isolator verbindet, zur Sterilitätsprüfung von radioaktiven Stoffen, bevorzugt radioaktiven Pharmazeutika und/oder Diagnostika.

Ein weiterer Aspekt der Erfindung betrifft ein Verfahren zur Sterilitätsprüfung von radioaktiven Stoffen, umfassend die Schritte
a) Membranfiltration einer Lösung eines radioaktiven Stoffes mittels einer Vorrichtung zur Membranfiltration umfassend mindestens einen Membranfilter in einem Isolator und Absaugung durch eine Anlage umfassend
   - eine Filtratflasche aufweisend mindestens eine flüssigkeitsfördernde Leitung und mindestens eine Druckausgleichsleitung, und
   - mindestens einen Transportwagen, wobei der Transportwagen geeignet ist, die Filtratflasche aufzunehmen,
      wobei der Transportwagen bewegbar ist,
      wobei mindestens die Filtratflasche von einer Abschirmung gegen ionisierende Strahlung umgeben ist,
   - eine Vakuumpumpe, wobei die Vakuumpumpe an die flüssigkeitsfördernde Leitung angeschlossen und geeignet ist, Filtrat enthaltend den radioaktiven Stoff in die Filtratflasche zu pumpen,
   - mindestens ein selbstabdichtendes Verbindungsstück, und
   - einen Isolator, wobei der Isolator mit der Filtratflasche auf dem Transportwagen über die mindestens eine flüssigkeitsfördernde Leitung und die mindestens eine Druckausgleichsleitung verbunden ist,
   wobei das mindestens eine selbstabdichtende Verbindungsstück die Filtratflasche über die flüssigkeitsfördernde Leitung und/oder die Druckausgleichsleitung mit dem Isolator verbindet,
b) Kultivierung der Membranfilter in einem Kulturmedium und
c) Prüfung des Kulturmediums auf Mikroorganismen.

In Ausführungsformen erfolgt das Verfahren in der Reihenfolge der Schritte a), b) und c).

Vorteilhaft werden durch die Membranfiltration selektiv Mikroorganismen von der Lösung eines radioaktiven Stoffes abgetrennt und bevorzugt wird der radioaktive Stoff mit dem Filtrat abgesaugt. Weiterhin vorteilhaft wird durch das erfindungsgemäße Verfahren bzw. die Verwendung der erfindungsgemäßen Anlage eine einfache und sichere Handhabung der radioaktiven Stoffe gewährleistet.

Zweckmäßig erfolgt die Durchführung des erfindungsgemäßen Verfahrens unter Verwendung von sterilen Materialien, insbesondere eines sterilen Membranfilters und sterilem Kulturmedium, und einem sterilisierten Isolator.

In Ausführungsformen ist die Lösung eines radioaktiven Stoffes eine wässrige Lösung, alkoholische Lösung und/oder Öl-basierte Lösung.

In Ausführungsformen wird die Lösung eines radioaktiven Stoffes durch Lösen mindestens eines festen oder pastösen radioaktiven Stoffes mit einem Lösungsmittel, insbesondere mit einem wässrigen, alkoholischen und/oder Carbonsäureester-basierten Lösungsmittel; erhalten.

In Ausführungsformen wird die Lösung eines radioaktiven Stoffes durch Verdünnen einer Öl-basierten Lösung eines radioaktiven Stoffes mit einem Lösungsmittel, insbesondere mit einem wässrigen, alkoholischen und/oder Carbonsäureester-basierten Lösungsmittel; erhalten.

Zweckmäßig weisen das Lösungsmittel keine antimikrobielle Eigenschaft auf.

Bevorzugt erfolgt das Lösen eines festen oder pastösen radioaktiven Stoffes oder das Verdünnen einer Öl-basierten Lösung mit Isopropylmyristat.

In Ausführungsformen erfolgt die Membranfiltration in Schritt a) mit mindestens einem Membranfilter aus Celluloseacetat oder Cellulosenitrat und/oder aufweisend Poren im Bereich von 0,2 µm bis 0,45 µm.

In Ausführungsformen erfolgt die Membranfiltration in Schritt a) einer wässrigen Lösung, einer schwach alkoholischen Lösung oder einer Öl-basierten Lösung mit mindestens einem Filter aus Cellulosenitrat.

In Ausführungsformen erfolgt die Membranfiltration in Schritt a) einer stark alkoholischen Lösung mit mindestens einem Filter aus Celluloseacetat.

In Ausführungsformen erfolgt die Membranfiltration in Schritt a) unter einem geringen Unterdruck. Unter einem geringen Unterdruck wird ein Druck im Bereich von 5 kPa bis 30 kPa verstanden. Vorteilhaft wird durch den geringen Unterdruck das Austreten von Stoffen aus dem Isolator verhindert.

In Ausführungsformen umfasst das erfindungsgemäße Verfahren weiterhin nach Schritt a) mindestens einen Spülschritt, wobei der Membranfilter mit einem Lösungsmittel, insbesondere einem wässrigen, alkoholischen und/oder Carbonsäureester-basierten Lösungsmittel; mindestens einmal gespült wird. In bevorzugten Ausführungsformen umfasst das erfindungsgemäße Verfahren weiterhin nach Schritt a) einen bis fünf Spülschritte, besonders bevorzugt drei Spülschritte.

In Ausführungsformen erfolgt die Kultivierung der Membranfilter in einem Kulturmedium in Schritt b) in einem Brutschrank.

Unter dem Begriff "Kulturmedium" wird ein flüssiges oder festes Medium verstanden, welches der Anzucht bzw. Kultivierung von Mikroorganismen dient.

In Ausführungsformen erfolgt die Kultivierung der Membranfilter in Schritt b) auf einem festen Kulturmedium (Nährboden) oder in einem flüssigen Kulturmedium (Nährmedium).

In bevorzugten Ausführungsformen erfolgt die die Kultivierung der Membranfilter in Schritt b) auf Agarplatten.

Unter dem Begriff "Brutschrank" oder auch "Inkubator" wird ein einstellbares Temperiergerät verstanden, welches fähig ist, konstante Luftfeuchtigkeits- und Temperatur-Bedingungen im Innenraum aufrechtzuerhalten.

In Ausführungsformen erfolgt die Kultivierung der Membranfilter in Schritt b) bei einer Temperatur im Bereich von 20 °C bis 37 °C, bevorzugt bei einer Temperatur im Bereich von 20 °C bis 25 °C oder im Bereich von 30 °C bis 35 °C.

In Ausführungsformen erfolgt die Kultivierung der Membranfilter in Schritt b) bei einer Luftfeuchtigkeit im Bereich von 30 % bis 70 %.

In Ausführungsformen erfolgt die Kultivierung der Membranfilter in Schritt b) unter aeroben und/oder anaeroben Bedingungen.

In Ausführungsformen erfolgt die Kultivierung der Membranfilter in Schritt b) für eine Dauer von mindestens 14 Tagen, bevorzugt für 14 Tage.

In Ausführungsformen erfolgt die Prüfung des Kulturmediums auf Mikroorganismen in Schritt c) durch visuelle Feststellung einer Koloniebildung auf einem festen Kulturmedium.

In alternativen Ausführungsformen erfolgt die Prüfung des Kulturmediums auf Mikroorganismen in Schritt c) durch visuelle Feststellung einer Trübung in einem flüssigen Kulturmedium.

In Ausführungsformen erfolgt die Prüfung des Kulturmediums auf Mikroorganismen in Schritt c) mindestens einmal, bevorzugt nach einer Kultivierung der Membranfilter für eine Dauer von 14 Tagen.

In weiteren Ausführungsformen erfolgt die Prüfung des Kulturmediums auf Mikroorganismen in Schritt c) mindestens zweimal, bevorzugt nach einer Kultivierung der Membranfilter für eine Dauer von 7 Tagen und von 14 Tagen.

In Ausführungsformen umfasst das erfindungsgemäße Verfahren weiterhin das Absaugen der Lösung eines radioaktiven Stoffes aus der Filtratflasche und Abklingen der Lösung eines radioaktiven Stoffes in einem Abklingbecken.

In Ausführungsformen umfasst das erfindungsgemäße Verfahren weiterhin die Sterilisierung des Isolators vor dem Schritt a) und/oder nach dem Schritt c). In Ausführungsformen erfolgt die Sterilisierung des Isolators durch Begasung mit Wasserstoffperoxid. Vorteilhaft erfolgt durch die Begasung mit Wasserstoffperoxid eine flächendeckende Dekontamination aller in den Arbeitsbereich bzw. in die Schleuse eingebrachten Materialien.

Zweckmäßig umfasst die Sterilisierung des Isolators durch Begasung mit Wasserstoffperoxid die anschließende Wiederherstellung einer Wasserstoffperoxid-freien Atmosphäre, so dass eine Prüfung auf Sterilität unter Ausschluss falsch-negativer Ergebnisse erfolgen kann.

In Ausführungsformen umfasst das erfindungsgemäße Verfahren weiterhin den Vergleich mit geeigneten Kontrollen, insbesondere mindestens einer Positivkontrolle, mindestens einer Negativkontrolle und/oder mindestens einer Umfeldkontrolle.

Unter dem Begriff "Positivkontrolle" wird eine Probe umfassend vermehrungsfähige Mikroorganismen verstanden, welche nach dem erfindungsgemäßen Verfahren ein "positives Ergebnis", d. h. ein Wachstum von Mikroorganismen; zeigt. Vorteilhaft ermöglichen Positivkontrollen den Ausschluss von Falsch-negativen Ergebnissen.

In Ausführungsformen umfasst die Positivkontrolle Kulturmedium und vermehrungsfähige Mikroorganismen.

Unter dem Begriff "Negativkontrolle" wird eine Probe verstanden, welche nach dem erfindungsgemäßen Verfahren, insbesondere die Schritte a), b) und c); ein "negatives Ergebnis", d. h. kein Wachstum von Mikroorganismen; zeigt. Vorteilhaft ermöglichen Negativkontrollen den Ausschluss von Falsch-positiven Ergebnissen.

In Ausführungsformen ist die Negativkontrolle Kulturmedium ohne Zugabe von Mikroorganismen.

Unter dem Begriff "Umfeldkontrolle" wird eine Probe verstanden, welche die Qualität der Luft und der Oberflächen der erfindungsgemäßen Anlage zeigt

Zweckmäßig erfolgt der Vergleich mit geeigneten Kontrollen, bevorzugt der mindestens einen Negativkontrolle und/oder der mindestens einen Umfeldkontrolle; bei einer erstmaligen Durchführung des erfindungsgemäßen Verfahrens und/oder bei einer Veränderung der Kultivierungsbedingungen.

Zweckmäßig erfolgt der Vergleich mit geeigneten Kontrollen, bevorzugt mit der mindestens einen Positivkontrolle; mit jeder Probe, d.h. der Lösung eines radioaktiven Stoffes.

Für die Realisierung der Erfindung ist es auch zweckmäßig, die vorbeschriebenen Ausführungsformen und Merkmale der Ansprüche zu kombinieren, insbesondere die Merkmale der erfindungsgemäßen Anlage auf die erfindungsgemäße Verwendung und das erfindungsgemäße Verfahren anzuwenden.

### Ausführungsbeispiele

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

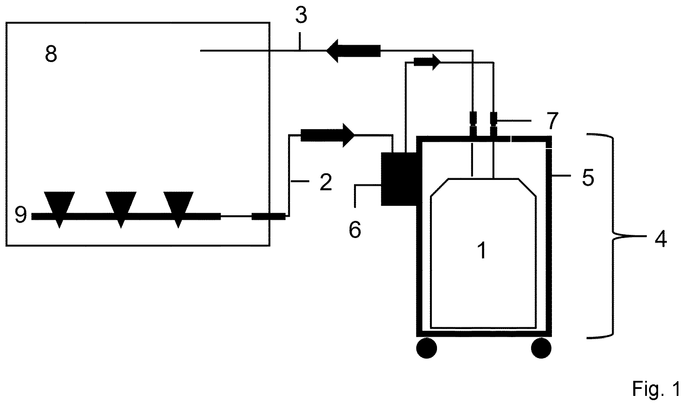

**Fig. 1** zeigt eine schematische Darstellung der erfindungsgemäßen Anlage zur Sterilitätsprüfung von radioaktiven Stoffen umfassend eine Filtratflasche **1,** aufweisend eine flüssigkeitsfördernde Leitung **2** und eine Druckausgleichsleitung **3,** einen bewegbaren Transportwagen **4,** wobei sich die Filtratflasche **1** innerhalb des bewegbaren Transportwagens **4** befindet und wobei der bewegbare Transportwagen **4** an der Unterseite vier Rollen aufweist. Das Gehäuse des Transportwagens **4** stellt eine Abschirmung gegen ionisierende Strahlung **5** dar. Der Transportwagen weist weiterhin eine Vakuumpumpe **6** auf. Die flüssigkeitsfördernde Leitung **2** und die Druckausgleichsleitung **3** weisen ein selbstabdichtendes Verbindungsstück **7** auf und sind mit dem Isolator **8** verbunden, insbesondere ist die flüssigkeitsfördernde Leitung **2** mit einer Vorrichtung zur Membranfiltration **9** innerhalb des Isolators **8** verbunden.

### Ausführungsbeispiel

### Sterilprüfung einer ¹⁷⁷Lu-DOTA-TOC-Lösung mittels einer erfindungsgemäßen Anlage

Eine ¹⁷⁷Lu-DOTA-TOC-Lösung mit einer Aktivität von ca. 7.000 MBq und der folgenden Zusammensetzung:
- 13 mg Natriumascorbat
- 31 mg Natriumacetat
- 4 mg 2,5-Dihydroxybenzoesäure
- 6 ml 0,04 M Essigsäure
- 10 ml 0,9 % Natriumchloridlösung
- 115 µg ¹⁷⁷Lu-DOTA-TOC in 115 µl 0,04 M Essigsäure,
wird in dem Isolator, insbesondere die Sterilbox, eingeschleust und ganz oder teilweise über Membranfilter (Cellulosenitrat, Porengröße 0,45µm) filtriert. Anschließend wird der Membranfilter mit 50 ml bis 300 ml Puffer gespült. Der Membranfilter wird danach jeweils in 100 ml Casein-Sojapepton-Bouillon und 100 ml Thioglykolat-Bouillon gegeben. Die Inkubation der Membranfilter in dem Kulturmedium erfolgt für 14 Tage, in Casein-Sojapepton-Bouillon bei einer Temperatur im Bereich von 20 °C bis 25 °C und in Thioglykolat-Bouillon bei einer Temperatur im Bereich von 30 °C bis 35 °C.

Die Auswertung erfolgt die visuelle Beurteilung der Trübung. Im Falle von einem Ausbleiben einer Trübung ist die Probe negativ für Mikroorganismenwachstum und somit steril. Im Falle einer Trübung ist die Probe positiv für Mikroorganismenwachstum und somit nicht steril.

Die mittels der erfindungsgemäßen Anlage abgepumpte Probenlösung in der Filtratflasche umfassend ¹⁷⁷Lu-DOTA-TOC wird mit dem Transportwagen zu einem Abklingbecken transportiert und nach dem Anschluss der flüssigkeitsfördernden Leitung an das Abklingbecken abgepumpt. Die Lösung wird im Abklingbecken bis zum Abklingen unterhalb der Freigrenze für mindestens 60 Tage belassen.

### Bezugszeichen

- 1: Filtratflasche
- 2: Flüssigkeitsfördernde Leitung
- 3: Druckausgleichsleitung
- 4: Transportwagen
- 5: Abschirmung gegen ionisierende Strahlung
- 6: Vakuumpumpe
- 7: Selbstabdichtendes Verbindungsstück
- 8: Isolator
- 9: Vorrichtung zur Membranfiltration

## Patentansprüche

1. Anlage zur Sterilitätsprüfung von radioaktiven Stoffen, umfassend
i. eine Filtratflasche (1) aufweisend mindestens eine flüssigkeitsfördernde Leitung (2) und mindestens eine Druckausgleichsleitung (3), und
ii. mindestens einen Transportwagen (4), wobei der Transportwagen (4) geeignet ist, die Filtratflasche (1) aufzunehmen,
wobei der Transportwagen (4) bewegbar ausgebildet ist,
wobei mindestens die Filtratflasche (1) von einer Abschirmung gegen ionisierende Strahlung (5) umgeben ist,
iii. eine Vakuumpumpe (6),
wobei die Vakuumpumpe (6) an die flüssigkeitsfördernde Leitung (2) angeschlossen und geeignet ist, Filtrat enthaltend den radioaktiven Stoff in die Filtratflasche (1) zu pumpen,
iv. mindestens ein selbstabdichtendes Verbindungsstück (7), und
v. einen Isolator (8), wobei der Isolator (8) mit der Filtratflasche (1) auf dem Transportwagen (4) über die mindestens eine flüssigkeitsfördernde Leitung (2) und die mindestens eine Druckausgleichsleitung (3) verbunden ist,
wobei der Isolator (8) eine Vorrichtung zur Membranfiltration (9) umfasst, wobei das mindestens eine selbstabdichtende Verbindungsstück (7) die Filtratflasche über die flüssigkeitsfördernde Leitung (2) und/oder die Druckausgleichsleitung (3) mit dem Isolator (8) verbindet.

2. Anlage nach Anspruch 1, **dadurch gekennzeichnet, dass** die Abschirmung gegen ionisierende Strahlung (5) eine Metallschicht und eine Plexiglasschicht aufweist.

3. Anlage nach Anspruch 1 oder 2, **dadurch gekennzeichnet, dass** die Abschirmung gegen ionisierende Strahlung (5) ein Metall mit einer Dicke im Bereich von 40 mm bis 100 mm aufweist.

4. Anlage nach einem der Ansprüche 1 bis 3, **dadurch gekennzeichnet, dass** die Filtratflasche aus Glas, Kunststoff und/oder Metall ist.

5. Anlage nach einem der Ansprüche 1 bis 4, **dadurch gekennzeichnet, dass** die Filtratflasche (1) einen Flüssigkeitsfüllstandmesser aufweist, wobei der Flüssigkeitsfüllstandmesser ein optisches und/oder akustisches Signal bei einem vordefinierten Füllstand des Filtrats abgibt.

6. Anlage nach einem der Ansprüche 1 bis 5, **dadurch gekennzeichnet, dass** die flüssigkeitsfördernde Leitung und die mindestens eine Druckausgleichsleitungaus Gummi und/oder Metall sind.

7. Anlage nach einem der Ansprüche 1 bis 6, **dadurch gekennzeichnet, dass** die Verbindung zwischen dem Isolator (8) mit der Filtratflasche (1) auf dem Transportwagen als Strahlenfalle ausgeführt ist.

8. Anlage nach einem der Ansprüche 1 bis 7, **dadurch gekennzeichnet, dass** die Vorrichtung zur Membranfiltration (9) mindestens einen Membranfilter aus Celluloseacetat oder Cellulosenitrat und/oder aufweisend Poren im Bereich von 0,2 bis 0,45 µm umfasst.

9. Verwendung einer Anlage nach einem der Ansprüche 1 bis 8 zur Sterilitätsprüfung von radioaktiven Stoffen, bevorzugt radioaktiven Pharmazeutika und/oder Diagnostika.

10. Verfahren zur Sterilitätsprüfung von radioaktiven Stoffen unter Verwendung einer Anlage nach einem der Ansprüche 1 bis 8 umfassend die Schritte
a) Membranfiltration einer Lösung eines radioaktiven Stoffes mittels einer Vorrichtung zur Membranfiltration umfassend mindestens einen Membranfilter in einem Isolator (8) und Absaugung durch eine Anlage umfassend
- eine Filtratflasche (1) aufweisend mindestens eine flüssigkeitsfördernde Leitung (2) und mindestens eine Druckausgleichsleitung (3), und
- mindestens einen Transportwagen (4), wobei der Transportwagen (4) geeignet ist, die Filtratflasche (4) aufzunehmen,
wobei der Transportwagen (4) bewegbar ist,
wobei mindestens die Filtratflasche (1) von einer Abschirmung gegen ionisierende Strahlung (5) umgeben ist,
- eine Vakuumpumpe (6), wobei die Vakuumpumpe (6) an die flüssigkeitsfördernde Leitung (2) angeschlossen und geeignet ist, Filtrat enthaltend den radioaktiven Stoff in die Filtratflasche (1) zu pumpen,
- mindestens ein selbstabdichtendes Verbindungsstück (7), und
- einen Isolator (8), wobei der Isolator (8) mit der Filtratflasche (1) auf dem Transportwagen (4) über die mindestens eine flüssigkeitsfördernde Leitung (2) und die mindestens eine Druckausgleichsleitung (3) verbunden ist,
wobei das mindestens eine selbstabdichtende Verbindungsstück (7) die Filtratflasche über die flüssigkeitsfördernde Leitung (2) und/oder die Druckausgleichsleitung (3) mit dem Isolator (8) verbindet,
b) Kultivierung der Membranfilter in einem Kulturmedium und
c) Prüfung des Kulturmediums auf Mikroorganismen.

11. Verfahren nach Anspruch 10, **dadurch gekennzeichnet, dass** die Lösung eines radioaktiven Stoffes eine wässrige Lösung, alkoholische Lösung und/oder Öl-basierte Lösung ist.

12. Verfahren nach Anspruch 10 oder 11, **dadurch gekennzeichnet, dass** die Kultivierung der Membranfilter in Schritt b) bei einer Temperatur im Bereich von 20 °C bis 37 °C, bei einer Luftfeuchtigkeit im Bereich von 30 % bis 70 % und/oder für eine Dauer von 14 Tagen erfolgt.

13. Verfahren nach einem der Ansprüche 10 bis 12 umfassend weiterhin das Absaugen der Lösung eines radioaktiven Stoffes aus der Filtratflasche und Abklingen der Lösung eines radioaktiven Stoffes in einem Abklingbecken.

## Claims

1. System for testing the sterility of radioactive substances, comprising
i. a filtrate bottle (1) comprising at least one liquid-conveying line (2) and at least one pressure-equalising line (3), and
ii. at least one transport carriage (4), wherein the transport carriage (4) is suitable for receiving the filtrate bottle (1), wherein the transport carriage (4) is movable, wherein at least the filtrate bottle (1) is surrounded by a shield against ionising radiation (5),
iii. a vacuum pump (6), wherein the vacuum pump (6) is connected to the liquid-conveying line (2) and is suitable for pumping filtrate containing the radioactive substance into the filtrate bottle (1),
iv. at least one self-sealing connection piece (7), and
v. an isolator (8), wherein the isolator (8) is connected to the filtrate bottle (1) on the transport carriage (4) via the at least one liquid-conveying line (2) and the at least one pressure-equalising line (3), wherein the isolator (8) comprises a device for membrane filtration (9), wherein the at least one self-sealing connection piece (7) connects the filtrate bottle to the isolator (8) via the liquid-conveying line (2) and/or the pressure-equalising line (3).

2. System according to claim 1, **characterised in that** the shield against ionising radiation (5) comprises a metal layer and a Plexiglas layer.

3. System according to either claim 1 or claim 2, **characterised in that** the shield against ionising radiation (5) comprises a metal having a thickness in the range of 40 mm to 100 mm.

4. System according to any of claims 1 to 3, **characterised in that** the filtrate bottle is made of glass, plastics material and/or metal.

5. System according to any of claims 1 to 4, **characterised in that** the filtrate bottle (1) comprises a liquid fill level meter, wherein the liquid fill level meter emits an optical and/or acoustic signal at a predefined fill level of the filtrate.

6. System according to any of claims 1 to 5, **characterised in that** the liquid-conveying line and the at least one pressure-equalising line are made of rubber and/or metal.

7. System according to any of claims 1 to 6, **characterised in that** the connection between the isolator (8) and the filtrate bottle (1) on the transport carriage is designed as a beam trap.

8. System according to any of claims 1 to 7, **characterised in that** the device for membrane filtration (9) comprises at least one membrane filter made of cellulose acetate or cellulose nitrate and/or having pores in the range of from 0.2 to 0.45 µm.

9. Use of a system according to any of claims 1 to 8 for testing the sterility of radioactive substances, preferably radioactive pharmaceuticals and/or diagnostic agents.

10. Method for testing the sterility of radioactive substances using a system according to any of claims 1 to 8, comprising the steps of
a) membrane filtration of a solution of a radioactive substance by means of a device for membrane filtration comprising at least one membrane filter in an isolator (8), and suction through a system comprising
- a filtrate bottle (1) comprising at least one liquid-conveying line (2) and at least one pressure-equalising line (3), and
- at least one transport carriage (4), wherein the transport carriage (4) is suitable for receiving the filtrate bottle (4), wherein the transport carriage (4) is movable,
wherein at least the filtrate bottle (1) is surrounded by a shield against ionising radiation (5),
- a vacuum pump (6), wherein the vacuum pump (6) is connected to the liquid-conveying line (2) and is suitable for pumping filtrate containing the radioactive substance into the filtrate bottle (1),
- at least one self-sealing connection piece (7), and
- an isolator (8), wherein the isolator (8) is connected to the filtrate bottle (1) on the transport carriage (4) via the at least one liquid-conveying line (2) and the at least one pressure-equalising line (3), wherein the at least one self-sealing connection piece (7) connects the filtrate bottle to the isolator (8) via the liquid-conveying line (2) and/or the pressure-equalising line (3),
b) culturing the membrane filters in a culture medium, and
c) testing the culture medium for microorganisms.

11. Method according to claim 10, **characterised in that** the solution of a radioactive substance is an aqueous solution, alcoholic solution and/or oilbased solution.

12. Method according to claim 10 or 11, **characterised in that** the culturing of the membrane filters in step b) is carried out at a temperature in the range of 20°C to 37°C, at an air humidity in the range of from 30% to 70% and/or for a duration of 14 days.

13. Method according to any of claims 10 to 12, further comprising sucking the solution of a radioactive substance from the filtrate bottle and decaying the solution of a radioactive substance in a decay tank.

## Revendications

1. Installation permettant de vérifier la stérilité de matières radioactives, comprenant
i. une bouteille de filtrat (1) présentant au moins une conduite d'alimentation en liquide (2) et au moins une conduite d'équilibrage de pression (3), et
ii. au moins un chariot de transport (4), le chariot de transport (4) étant adapté pour recevoir la bouteille de filtrat (1), dans laquelle le chariot de transport (4) est conçu de manière à pouvoir être déplacé, dans laquelle au moins la bouteille de filtrat (1) est entourée d'un blindage contre le rayonnement ionisant (5),
iii. une pompe à vide (6), dans laquelle la pompe à vide (6) est raccordée à la conduite d'alimentation en liquide (2) et adaptée pour pomper du filtrat contenant la matière radioactive dans la bouteille de filtrat (1),
iv. au moins une pièce de liaison auto-étanche (7), et
v. un isolateur (8), dans laquelle l'isolateur (8) est relié à la bouteille de filtrat (1) sur le chariot de transport (4) par l'intermédiaire de l'au moins une conduite d'alimentation en liquide (2) et de l'au moins une conduite d'équilibrage de pression (3), dans laquelle l'isolateur (8) comprend un dispositif permettant la filtration sur membrane (9), dans laquelle l'au moins une pièce de liaison auto-étanche (7) relie la bouteille de filtrat à l'isolateur (8) par l'intermédiaire de la conduite d'alimentation en liquide (2) et/ou de la conduite d'équilibrage de pression (3).

2. Installation selon la revendication 1, **caractérisée en ce que** le blindage contre le rayonnement ionisant (5) présente une couche de métal et une couche de plexiglas.

3. Installation selon la revendication 1 ou 2, **caractérisée en ce que** le blindage contre le rayonnement ionisant (5) présente un métal d'une épaisseur dans la plage de 40 mm à 100 mm.

4. Installation selon l'une des revendications 1 à 3, **caractérisée en ce que** la bouteille de filtrat est en verre, en plastique et/ou en métal.

5. Installation selon l'une des revendications 1 à 4, **caractérisée en ce que** la bouteille de filtrat (1) présente un indicateur de niveau de remplissage de liquide, dans laquelle l'indicateur de niveau de remplissage de liquide émet un signal optique et/ou acoustique pour un niveau de remplissage prédéfini du filtrat.

6. Installation selon l'une des revendications 1 à 5, **caractérisée en ce que** la conduite d'alimentation en liquide et l'au moins une conduite d'équilibrage de pression sont en caoutchouc et/ou en métal.

7. Installation selon l'une des revendications 1 à 6, **caractérisée en ce que** la liaison entre l'isolateur (8) et la bouteille de filtrat (1) sur le chariot de transport est réalisée en tant que piège de rayons.

8. Installation selon l'une des revendications 1 à 7, **caractérisée en ce que** le dispositif permettant la filtration sur membrane (9) comprend au moins un filtre à membrane en acétate de cellulose ou en nitrate de cellulose et/ou présentant des pores dans la plage de 0,2 à 0,45 µm.

9. Utilisation d'une installation selon l'une des revendications 1 à 8 pour la vérification de la stérilité de matières radioactives, de préférence de produits pharmaceutiques et/ou de produits diagnostiques radioactifs.

10. Procédé permettant la vérification de la stérilité de matières radioactives à l'aide d'une installation selon l'une des revendications 1 à 8, comprenant les étapes de
a) filtration sur membrane d'une solution d'une matière radioactive au moyen d'un dispositif permettant la filtration sur membrane comprenant au moins un filtre à membrane dans un isolateur (8) et aspiration par une installation comprenant
- une bouteille de filtrat (1) présentant au moins une conduite d'alimentation en liquide (2) et au moins une conduite d'équilibrage de pression (3), et
- au moins un chariot de transport (4), dans lequel le chariot de transport (4) est adapté pour recevoir la bouteille de filtrat (4), dans lequel le chariot de transport (4) est conçu de manière à pouvoir être déplacé,
dans lequel au moins la bouteille de filtrat (1) est entourée d'un blindage contre le rayonnement ionisant (5),
- une pompe à vide (6), dans lequel la pompe à vide (6) est raccordée à la conduite d'alimentation en liquide (2) et adaptée pour pomper du filtrat contenant la matière radioactive dans la bouteille de filtrat (1), au moins une pièce de liaison auto-étanche (7), et
- un isolateur (8), dans lequel l'isolateur (8) est relié à la bouteille de filtrat (1) sur le chariot de transport (4) par l'intermédiaire de l'au moins une conduite d'alimentation en liquide (2) et de l'au moins une conduite d'équilibrage de pression (3), dans lequel l'au moins une pièce de liaison auto-étanche (7) relie la bouteille de filtrat à l'isolateur (8) par l'intermédiaire de la conduite d'alimentation en liquide (2) et/ou de la conduite d'équilibrage de pression (3),
b) culture des filtres à membrane dans un milieu de culture et
c) vérification des micro-organismes dans le milieu de culture.

11. Procédé selon la revendication 10, **caractérisé en ce que** la solution d'une matière radioactive est une solution aqueuse, une solution alcoolique et/ou une solution à base d'huile.

12. Procédé selon la revendication 10 ou 11, **caractérisé en ce que** la culture des filtres à membrane à l'étape b) s'effectue à une température dans la plage de 20 °C à 37 °C, à une humidité dans la plage de 30 % à 70 % et/ou pour une durée de 14 jours.

13. Procédé selon l'une des revendications 10 à 12, comprenant en outre l'aspiration de la solution d'une matière radioactive hors de la bouteille de filtrat et la désactivation de la solution d'une matière radioactive dans une piscine de désactivation.
